# EUROPEAN PATENT APPLICATION

(11) **EP 2 075 577 A1**
(43) Date of publication of application: **01.07.2009**
(21) Application number: 08169262.6
(22) Date of filing: 17.11.2008
(51) Int. Cl.: G01N 27/407, G01N 33/00

(54) **Composition for use in a NOx electrode, method of making the same, an NOx electrode derived therefrom, and method of detecting NOx**

(30) Priority: 30.11.2007 US 948167
(71) Applicant: Delphi Technologies, Inc., Troy, Michigan 48007 (US)
(72) Inventor: Wang, Da Y., Troy, MI 48084 (US); Yao, Sheng, Macomb, MI 48044 (US); Briggs, Elizabeth M., Chesterfield, MI 48047-4303 (US); Bloink, Raymond L., Swartz Creek, MI 48473 (US); Cabush, David D., Howell, MI 48843 (US)
(74) Representative: Denton, Michael John

(57) **Abstract**

Disclosed herein is a composition for use in a NOx electrode comprising:

Tb(1-x)Ln(x)E(1-y)Q(y1)X(y2)Z(y3)O3

wherein Ln is a lanthanoid or a combination of lanthanoids, E is a metal selected from chromium, iron, and a combination thereof, Q is an element selected from magnesium, calcium, strontium, and a combination thereof, X is an element selected from boron, lead, phosphorus, germanium, and a combination thereof, Z is an element selected from barium, silicon, aluminum, and a combination thereof, x is from 0 to about 0.5, y is from about 0.05 to about 0.8, and y1, y2, y3 are independently from 0 to about 0.8, with the proviso that y = y1 + y2 + y3, and y2 + y3 is greater than 0. Also disclosed are a method of making it, electrodes and sensors comprising it, and a method of detecting NOx.

## Description

### BACKGROUND OF THE INVENTION

Combustion engines that run on fossil fuels generate exhaust gases, which contain undesirable pollutants. Illustrative undesirable pollutants include nitrogen oxide gases (NOx), unburned hydrocarbon gases (HC), and carbon monoxide gas (CO). The automotive industry uses exhaust gas sensors in automotive vehicles to sense the composition of the exhaust gases for pollution control. For example, HC emissions can be reduced using sensors that can sense the concentration of oxygen (O₂) in the exhaust gases for alteration and optimization of the air to fuel ratio for combustion.

Some automotive vehicles utilize various pollution-control after treatment devices such as NOx absorber(s), selective catalytic reduction (SCR) catalyst(s), and/or the like, to reduce NOx emissions. NOx reduction is accomplished by using ammonia gas (NH₃), which can be generated from the reaction of urea with steam. In order for SCR catalysts to function efficiently and to avoid pollution breakthrough, a feedback control system is used to manage the regeneration cycle of the NOx traps. NH₃ sensors are used for the feedback control system to be more effective. One group of NH₃ sensors operate based on the Nernst Principle, that is, the sensor converts chemical energy from NH₃ into electromotive force (emf). The sensor can measure this electromotive force to determine the partial pressure of NH₃ in a sample gas. However, such sensors also convert the chemical energy from NOx into emf. This undesirable sensing of NOx by the NH₃ sensor is corrected using NOx sensors. NOx sensors can sometimes suffer from slow and/or inconsistent response time, and/or from high impedance and/or inconsistent emf output, which hinder the efficiency of the NOx sensor.

Therefore, there exists a need for NOx sensors with faster and more consistent response time, low impedance, and consistent emf output.

### SUMMARY OF THE INVENTION

The above-described and other drawbacks can be addressed by a composition for use in a NOx electrode, comprising a material according to the Formula (I):

Tb₍₁₋ₓ₎Ln₍ₓ₎E_{(1-y)}Q_{(y1)}X_{(y2})Z_{(y3})O₃ (I)

wherein Ln is a lanthanoid selected from the group consisting of lanthanum, cerium, praseodymium, neodymium, promethium, samarium, europium, gadolinium, dysprosium, holmium, erbium, thulium, ytterbium, and a combination thereof, E is a metal selected from the group consisting of chromium, iron, and a combination thereof, Q is an element selected from the group consisting of magnesium, calcium, strontium, and a combination thereof, X is an element selected from the group consisting of boron, lead, phosphorus, germanium, and a combination thereof, Z is an element selected from the group consisting of barium, silicon, aluminum, and a combination thereof, x is from 0 to about 0.5, y is from about 0.05 to about 0.8, and y1, y2, y3 are independently from 0 to about 0.8, with the proviso that y = y1 + y2 + y3, and y2 + y3 is greater than 0.

In one embodiment, a NOx electrode comprises a composition, the composition comprising a material according to the Formula (I), wherein Ln is a lanthanoid selected from the group consisting of lanthanum, cerium, praseodymium, neodymium, promethium, samarium, europium, gadolinium, dysprosium, holmium, erbium, thulium, ytterbium, and a combination thereof, E is a metal selected from the group consisting of chromium, iron, and a combination thereof, Q is an element selected from the group consisting of magnesium, calcium, strontium, and a combination thereof, X is an element selected from the group consisting of boron, lead, phosphorus, germanium, and a combination thereof, Z is an element selected from the group consisting of barium, silicon, aluminum, and a combination thereof, x is from 0 to about 0.5, y is from about 0.05 to about 0.8, and y1, y2, y3 are independently from 0 to about 0.8, with the proviso that y = y1 + y2 + y3, and y2 + y3 is greater than 0.

In another embodiment, a NOx or NH₃ sensor element comprises a first NOx electrode, a reference electrode, and an electrolyte disposed between the first NOx electrode and the reference electrode, wherein the first NOx electrode comprises the material according to the Formula (I).

In another embodiment, a NOx or NH₃ sensor comprises a NOx sensor element, comprising a first NOx electrode, a reference electrode, and an electrolyte disposed between the first NOx electrode and the reference electrode, wherein the first NOx electrode comprises the material according to the Formula (I), a housing, wherein the NOx or NH₃ sensor element is disposed in the housing, and wherein the housing has an inlet for receiving a gas such that the gas can contact the sensor element, and a catalyst upstream of the sensor element such that the gas contacts the catalyst prior to contacting the sensor element, and undesirable pollutants such as hydrocarbons, carbon monoxide, ammonia, hydrogen, or a combination thereof, in the gas are converted to nitrogen, carbon dioxide, water, or a combination thereof.

Another embodiment is a method of detecting NOx in a gas, comprising contacting a first NOx electrode with the gas, determining a NOx emf between the first NOx electrode and a first reference electrode, and determining a NOx concentration using the NOx emf, wherein the first NOx electrode comprises the material according to the Formula (I).

Another embodiment is a method of making the material according to the Formula (I), comprising heating a mixture of an oxide of Tb, E, and X; Tb, E, and Z; or Tb, E, X, and Z; and, optionally, an oxide of Ln; Q; or Ln and Q at a temperature of about 500 °C to about 1600 °C.

### BRIEF DESCRIPTION OF THE DRAWINGS

Referring now to the drawings wherein like elements are numbered alike in several FIGURES:

FIG. 1 and FIG. 2 are an exploded view of an exemplary planar NOx sensor element;

FIG. 3 is sensor for placement in a gas stream ;

FIG. 4 is a graphical representation of the NOx response time at 570° C;

FIG. 5 is a graphical representation of the NOx response time at 531° C;

FIG. 6 is a graphical representation of the NOx response time at 570° C for TbCr_{0.8}Mg_{0.2}O₃; and

FIG. 7 is a graphical representation of the sensing behavior of a sensor comprising TbCr_{0.8}Mg_{0.1}B_{0.1}O₃ measured at five different temperatures by controlling the temperature sensor at five different resistance values (about 600°C, 570°C, 540°C, 510°C, 480°C).

### DESCRIPTION OF THE PREFERRED EMBODIMENTS

Surprisingly, the present inventors have discovered that a composition comprising a material having the Formula (I):

Tb₍₁₋ₓ₎Ln₍ₓ₎E_{(1-y)}Q_{(y1)}X_{(y2)}Z_{(y3)}O₃ (I)

can be used in NOx electrodes that exhibit faster and more consistent response time, low impedance, and consistent emf output when sensing NOx in comparison to a similar NOx electrode free of the composition comprising the material having the formula (I).

As used herein, chemical elements, for example terbium, can be represented by their full name, i.e., "terbium", or by their chemical symbol, i.e., "Tb".

Ln, as used in Formula (I), is a lanthanoid element other than terbium. Ln is selected from lanthanum, cerium, praseodymium, neodymium, promethium, samarium, europium, gadolinium, dysprosium, holmium, erbium, thulium, and ytterbium. A combination comprising at least one of the foregoing lanthanoid elements can also be used. In one embodiment, Ln is a lanthanoid element selected from lanthanum, cerium, erbium, and a combination thereof. In another embodiment, Ln is a lanthanoid element selected from lanthanum, cerium, and a combination thereof. In one advantageous embodiment, Ln is lanthanum.

E, as used in Formula (I), is selected from chromium and iron. A combination comprising at least one of the foregoing metals E can also be used. In one embodiment, E is iron. In one advantageous embodiment, E is chromium.

Q, as used in Formula (I), comprises an alkaline earth metal. Q comprises magnesium, calcium, or strontium. A combination comprising at least one of the foregoing elements Q is also within the scope of Formula (I). In one embodiment, Q is calcium. In another embodiment, Q is strontium. In one advantageous embodiment, Q is magnesium.

Formula (I) further comprises X. X comprises boron, lead, phosphorus, germanium, or a combination comprising at least one of the foregoing elements. In one embodiment, X comprises boron, lead, phosphorus, or a combination thereof. In another embodiment, X comprises boron, lead, or a combination thereof. In one advantageous embodiment, X is boron. In another advantageous embodiment, X is lead.

Z, as used in Formula (I), comprises barium, silicon, aluminum, or a combination comprising at least one of the foregoing elements. In one embodiment, Z comprises barium, silicon, or a combination thereof. In one advantageous embodiment, Z is barium.

According to Formula (I), x is from 0 to about 0.5. That is, Ln can be present in or can be absent from Formula (I). When Ln is present, that is, when x is greater than 0, it is also less than or equal to about 0.5. Specifically, x is from 0 to about 0.3, and more specifically, from 0 to about 0.1. In one embodiment, x is from 0 to about 0.5. In another embodiment, x is from about 0.001 to about 0.1. In one advantageous embodiment, x is 0.01.

y is from about 0.05 to about 0.8, and y1, y2, and y3 are independently from 0 to about 0.8, with the proviso that y = y1 + y2 + y3, and y2 + y3 is greater than 0. Therefore, E is always present in Formula (I), and so is one of X and Z. Thus, Formula (I) always comprises Tb, E, at least one of X and Z, and O. In one embodiment, y1 is 0, and y2 and y3 are both greater than 0. In another embodiment, y1 and y2 are 0, and y3 = y. In another embodiment, y1 and y3 are 0, and y2 = y. In yet another embodiment, y1, y2, and y3 are all greater than 0.

In one embodiment, y is from about 0.1 to about 0.7. In another embodiment embodiment, y is from about 0.2 to about 0.5. In one advantageous embodiment, y is about 0.2. In another advantageous embodiment, y is about 0.3. In yet another advantageous embodiment, y is about 0.5.

In one embodiment, y1 is from 0 to about 0.5. In another embodiment, y1 is from 0 to about 0.2. In one advantageous embodiment, y1 is less than 0.2. In another advantageous embodiment, y1 is greater than 0 and less than 0.2.

In one embodiment, y2 is from about 0.01 to about 0.5. In another embodiment, y2 is from about 0.01 to about 0.4. In an advantageous embodiment, y2 is from about 0.025 to about 0.4. In another advantageous embodiment, y2 is selected from 0.025, 0.05, 0.01, 0.1, 0.2, and 0.4.

In one embodiment, y3 is from 0 to about 0.5. In another embodiment, y3 is from 0 to about 0.2. In one advantageous embodiment, y3 is 0. In another advantageous embodiment, y3 is 0.2.

Thus, in one embodiment, Formula (I) comprises Tb, E, X, and O. In another embodiment, Formula (I) comprises Tb, E, Z, and O. In another embodiment, Formula (I) comprises Tb, E, X, Z, and O. In another embodiment, Formula (I) comprises Tb, Ln or Q, E, X or Z, and O. In another embodiment, Formula (I) comprises Tb, Ln, E, X, and O. In another embodiment, Formula (I) comprises Tb, Ln, E, Z, and O. In another embodiment, Formula (I) comprises Tb, Ln, E, X, Z, and O. In another embodiment, Formula (I) comprises Tb, Q, E, X, and O. In another embodiment, Formula (I) comprises Tb, Q, E, Z, and O. In another embodiment, Formula (I) comprises Tb, Q, E, X, Z, and O. In another embodiment, Formula (I) comprises Tb, Ln, Q, E, X, and O. In another embodiment, Formula (I) comprises Tb, Ln, Q, E, Z, and O. Yet in another embodiment, Formula (I) comprises Tb, Ln, Q, E, X, Z, and O.

In one advantageous embodiment, Formula (I) comprises Tb, E, at least one of X and Z, and at least one of Ln and Q. In a further advantageous embodiment, Formula (I) comprises Tb, Cr, Mg, X, and lanthanum. In an even further advantageous embodiment, Formula (I) consists of Tb, Cr, Mg, and X.

In another embodiment, E comprises chromium, Q comprises an element selected from the group consisting of magnesium, strontium, and a combination comprising at least one of the foregoing elements, X comprises an element selected from the group consisting of boron, lead, phosphorus, and a combination comprising at least one of the foregoing elements, and Z comprises an element selected from the group consisting of barium, silicon, and a combination comprising at least one of the foregoing elements.

Ln, Q, X, and Z affect, inter alia, the emf output, impedance, and response time of the NOx electrode. However, and not wishing to be bound by theory, it is believed that impedance is specifically affected by Ln and Z, response time is specifically affected by X, and emf output is specifically affected by Q. Therefore, one with ordinary skill in the art can determine the amount of Ln, Q, X, and Z in Formula (I) according to the desired properties of the NOx electrode.

Thus, if the impedance of a NOx electrode comprising Tb, E, and O is adversely high, the addition of Ln and/or Z can be advantageous. If the response time of a NOx electrode comprising Tb, E, and O is adversely slow, the addition of X can be advantageous. If the emf output of a NOx electrode comprising Tb, E, and O is adversely inconsistent, the addition of Q can be advantageous. However, the addition of Ln, Q, X, and/or Z can affect any or all of the emf output, impedance, and response time. For example, the addition of Q can further advantageously affect the response time of the electrode.

Non-limiting examples of materials according to Formula (I) include TbCr_{0.8}Mg_{0.15}B_{0.05}O₃, TbCr_{0.8}Mg_{0.1}B_{0.1}O₃, TbCr_{0.7}Mg_{0.1}B_{0.2}O₃, TbCr_{0.5}Mg_{0.1}B_{0.4}O₃, TbCr_{0.8}Mg_{0.19}Pb_{0.01}O₃, TbCr_{0.8}Mg_{0.15}Pb_{0.05}O₃, TbCr_{0.8}Mg_{0.15}P_{0.05}O₃, Tb_{0.99}La_{0.01}Cr_{0.8}Mg_{0.175}B_{0.025}O₃, TbCr_{0.8}Ba_{0.2}O₃, and TbCr_{0.8}Si_{0.2}O₃. In one advantageous embodiment, the material according to Formula (I) is selected from the group consisting of TbCr_{0.8}Mg_{0.15}B_{0.05}O₃, TbCr_{0.8}Mg_{0.1}B_{0.1}O₃, TbCr_{0.7}Mg_{0.1}B_{0.2}O₃, TbCr_{0.5}Mg_{0.1}B_{0.4}O₃, TbCr_{0.8}Mg_{0.15}Pb_{0.05}O₃, and a combination thereof.

The material according to Formula (I) can be prepared using any suitable method available to one with ordinary skill in the art. One method of preparing the material according to Formula (I) is by mixing the oxides of Tb, E, and X or Z, and if desired, the oxides of Ln and/or Q, and heating the resulting mixture to a desired temperature for a suitable time effective at producing the material according to Formula (I). The temperature and time required depends on the material according to Formula (I).

The temperature that the resulting mixture of oxides is heated to can be from about 100 °C to about 1600 °C, specifically from about 200 °C to about 1500 °C, more specifically from about 300 °C to about 1400 °C, more specifically from about 400 °C to about 1300 °C, more specifically from about 500 °C to about 1200 °C, more specifically from about 600 °C to about 1200 °C, more specifically from about 700 °C to about 1200 °C, and even more specifically from about 800 °C to about 1200 °C. In one embodiment, the temperature that the resulting mixture of oxides is heated to is from about 500 °C to about 1600 °C. In one advantageous embodiment, the temperature that the resulting mixture of oxides is heated to is about 1200 °C.

The time for which the resulting mixture of oxides is heated to can be from about 0.5 to about 48 hours. Specifically, the time can be from about 1 hour to about 40 hours, more specifically from about 1.5 hours to about 30 hours, more specifically from about 2 hours to about 20 hours, more specifically from about 2.5 hours to about 19 hours, more specifically from about 3 hours to about 18 hours, more specifically from about 3.5 hours to about 17 hours, more specifically from about 4 hours to about 16 hours, more specifically from about 4.5 hours to about 15 hours, more specifically from about 5 hours to about 14 hours, more specifically from about 5.5 hours to about 13 hours, more specifically from about 6 hours to about 12 hours, more specifically from about 6 hours to about 11 hours, more specifically from about 6 hours to about 10 hours, more specifically from about 6 hours to about 9 hours, and even more specifically from about 6 hours to about 8.5 hours. In one embodiment, the time for which the resulting mixture of oxides is heated to is from about 4 hours to about 10 hours, more specifically from about 5 hours to about 9 hours, more specifically from about 6 hours to about 8 hours, and even more specifically for about 7 hours. In an advantageous embodiment, the time is for about 6 hours.

Thus, for example, TbCr_{0.8}Mg_{0.15}B_{0.05}O₃, TbCr_{0.8}Mg_{0.1}B_{0.1}O₃, TbCr_{0.7}Mg_{0.1}B_{0.2}O₃, and TbCr_{0.5}Mg_{0.1}B_{0.4}O₃ can be prepared by heating a mixture of Tb₃O₄, Cr₂O₃, MgO, and B₂O₃ at about 1200 °C for about 6 hours, whereas TbCr_{0.8}Mg_{0.15}Pb_{0.05}O₃ can be prepared by heating a mixture of Tb₃O₄, Cr₂O₃, MgO, and Pb₂O at about 1200 °C for about 6 hours.

While the composition comprising the material of Formula (I) has been described in terms of Tb, Ln, E, Q, X, Z, and O, it is to be understood that it can further comprise any other element as long as the other elements do not adversely affect the function of the material. Other elements can include, for example, Ti, Ta, K, V, Ag, Cd, W, Sn, Mn, Ni, Zn, Na, Zr, Nb, Co, Rh, and combinations thereof. In one advantageous embodiment, the material of Formula (I) is free of other elements, that is, it consists of Tb, E, and X; Tb, E, and Z; or Tb, E, X, and Z; and, optionally, Ln; Q; or Ln and Q.

A NOx electrode advantageously comprises the composition comprising the material having the Formula (I). A NOx or NH₃ sensor element advantageously comprises the foregoing NOx electrode. Further, a NOx or NH₃ sensor advantageously comprises the NOx or NH₃ sensor element comprising the NOx electrode comprising the material having the Formula (I).

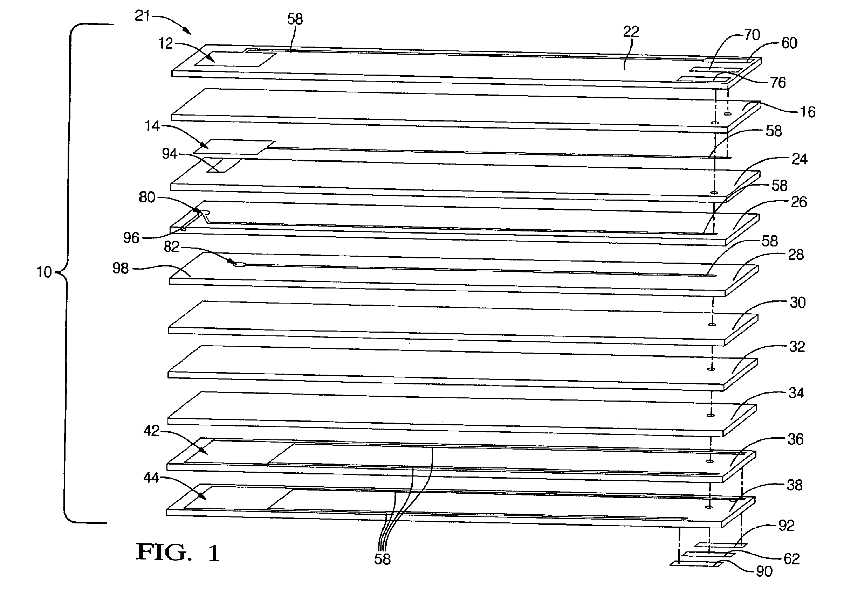

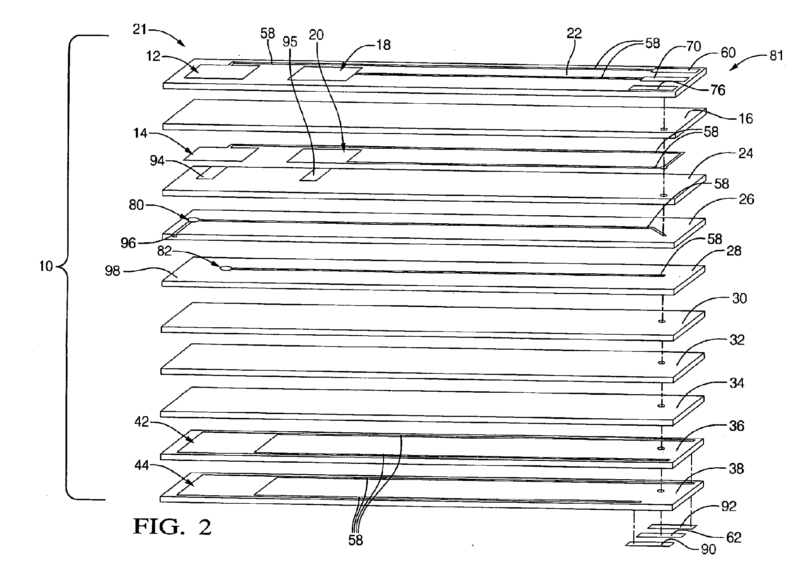

Thus, referring now to FIG. 1 and FIG. 2, exemplary sensor element 10 comprises a NOx sensing cell (12/16/14) comprising a first NOx electrode 12, a first reference electrode 14 and an electrolyte layer 16, and optionally (as shown in Figure 2) a second NOx sensing cell (18/16/20) comprising a second NOx electrode 18, a second reference electrode 20 and the electrolyte layer 16. The NOx sensing cells 12/16/14 and 18/16/20 are disposed at a sensing end 21 of the sensor element 10. The sensor element 10 comprises insulating layers 22, 24, 28, 34, 36, 38, and active layers, which include the electrolyte layer 16 and layers 26, 30, and 32. The active layers can conduct oxygen ions, where the insulating layers can insulate sensor components from electrical and ionic conduction. In an exemplary embodiment, the electrolyte layer 16 is disposed between insulating layers 22 and 24, active layer 26 is disposed between insulating layers 24 and 28, and active layers 30 and 32 are disposed between insulating layers 28 and 34.

The sensor element 10 can further comprise a temperature sensor (not shown), an air-fuel sensing cell comprising the active layer 26 along with an electrode 80 and an electrode 82 (80/26/82), a heater 44 disposed between the insulating layers 36 and 38, and an electromagnetic shield 42 (also known as a ground plane layer) disposed between the insulating layers 34 and 36. A first inlet 94 is defined by a first surface of the insulating layer 24 and by a surface of the electrolyte 16, proximate the first reference electrode 14. A second inlet 95 is defined by a first surface of the insulating layer 24 and by a surface of the electrolyte 16, proximate the second reference electrode 20. A third inlet 96 is defined by a first surface of the active layer 26 and a second surface of the insulating layer 24, proximate the electrode 80. A fourth inlet 98 is defined by a first surface of the active layer 26 and a second surface of the insulating layer 24, proximate the electrode 80. In addition, the sensor element 10 comprises electrical leads (58), contact pads (60, 64, 70, 76, 90, 92), additional ground plane layer(s) (not shown), and the like.

The first and second NOx electrodes 12, 18 are disposed in physical and ionic communication with electrolyte 16 and can be disposed in fluid communication with a sample gas (e.g., a gas being monitored or tested for its NOx concentration). The electrode materials have NOx sensing capability (e.g., interacting NOx gas to produce an emf), electrical conducting capability (conducting electrical current produced by the emf), and gas diffusion capability (providing sufficient open porosity so that gas can diffuse throughout the electrode and to the interface region of the electrodes 12,18 and electrolyte 16). The NOx electrode 12 comprises the material having the Formula (I). The NOx electrode 18 can comprise the material having the Formula (I), or another material with the above properties at the selected operating temperatures. These materials include YbCrO₃, LaCrO₃, ErCrO₃, EuCrO₃, SmCrO₃, HoCrO₃, GdCrO₃, NdCrO₃, TbCrO₃, ZnFe₂O₄, MgFe₂O₄, ZnCr₂O₄, and a combination comprising at least one of the foregoing materials. In an advantageous embodiment, the NOx electrode 18 comprises the material having the Formula (I).

The NOx electrodes can be formed to operate at desirable temperatures, depending upon the gas to be sensed. For example, the NOx electrode can operate at a temperature of about 200°C to about 400°C. In other embodiments, the NOx electrode can operate at a temperature of about 500°C to about 650°C, a temperature of about 650°C to about 700°C, or a temperature of greater than or equal to about 700°C. The operating temperature of the electrodes can be tailored with the use of Ln, E, Q, X, and/or Z in Formula (I), and/or with the use of other dopant(s). Non-limiting examples of other dopants include Ti, Ta, K, V, Ag, Cd, W, Sn, Mn, Ni, Zn, Na, Zr, Nb, Co, and Rh, as well as combinations comprising at least one of the foregoing other dopants.

In addition to the temperature, the contact resistance of the electrodes in contact with the electrolyte can be adjusted/controlled, e.g., so that the contact resistance is at least within the same order of magnitude of the electrolyte itself to enable the emf to be measured mostly by external measurement devices (such as voltmeters) without much internal drop of emf voltage at the contact area. An example is shown in Table 1 below.

The reference electrodes 14, 20 are disposed in physical contact and in ionic communication with the electrolyte 16, and can be disposed in fluid communication with the sample gas or reference gas; preferably with the sample gas. The materials comprising the reference electrodes have oxygen catalyzing capability (e.g., catalyzing equilibrium O₂ gas to produce an emf), electrical conducting capability (conducting electrical current produced by the emf), and/or gas diffusion capability (providing sufficient open porosity so that gas can diffuse throughout the electrode and to the interface region of the reference electrode 14 or 20 and electrolyte 22). Suitable reference electrode materials include Pt, Pd, Os, Rh, Ir, Au, Ru, and the like, as well as mixtures comprising at least one of the foregoing materials. The reference electrode can further include metal oxides such as zirconia and alumina that can increase the reference electrode porosity and increase the contact area between the electrode and the electrolyte.

The electrolyte layer 16 has oxygen ion conducting and fluid separation (limiting fluid communication of the sample gases on each side of the electrolyte layer 16) capabilities. The electrolyte layer 16 can be any size capable of providing sufficient ionic communication for the NOx sensing cell (12/16/20) or cells (12/16/20 and 18/16/20). The electrolyte layer 16 can be the entire length and width of the sensor element 10 or portions thereof. Possible electrolyte layer materials include zirconia, ceria, calcium oxide, yttria, lanthanum oxide, magnesia, alumina, indium oxide and the like, as well as combinations comprising at least one of the foregoing electrolyte materials, such as yttria doped zirconia, LaGaO₃, SrCeO₃, BaCeO₃, and CaZrO₃.

The air-fuel sensing cell (80/26/82) can detect the air to fuel ratio of the sample gas. When a constant potential is applied to electrodes 80 and 82, the current through the air-fuel sensing cell 80/26/82 is limited by the oxygen available in the inlets 96, 98 and at the electrodes 80, 82. Therefore, by measuring the potential at the air-fuel sensing cell 80/26/82, the processor can determine the air-to-fuel ratio of the gas. This same cell can also be used for sensing the temperature of the gas. In this mode an AC signal will be applied to the electrode 80 and 82, and the impedance of the electrolyte 26 between the two electrodes 80 and 82 is used for temperature determination.

The heater 44 can be employed to maintain the sensor element 10 at a selected operating temperature. The heater 44 can be positioned as part of the monolithic design of the sensor element 10, for example between insulating layer 36 and insulating layer 38, in thermal communication with the air-fuel sensing cell 80/26/82 and the sensing cells 12/16/14, 18/16/20. In other embodiments, the heater could be in thermal communication with the cells without necessarily being part of a monolithic laminate structure with them, e.g., simply by being in close physical proximity to a cell. More specifically, the heater can be capable of maintaining the sensing end 21 of the sensor element 10 at a sufficient temperature to facilitate the various electrochemical reactions therein. The heater can be a resistance heater and can comprise a line pattern (connected parallel lines, serpentine, and/or the like). The heater can comprise, for example, platinum, aluminum, palladium, and the like, as well as combinations comprising at least one of the foregoing, oxides comprising at least one of the foregoing metals. Contact pads, for example, the fourth contact pad 66 and the fifth contact pad 68, can transfer current to the heater from an external power source.

A temperature sensor (not shown) comprises any temperature sensor capable of monitoring the temperature of the sensing end 21 of the sensor element 10, such as, an impedance-measuring device or a metal-like resistance-measuring device. In an illustrative embodiment, a metal-like resistance temperature sensor can comprise, for example, a line pattern (connected parallel lines, serpentine, and/or the like). Some possible materials include, but are not limited to, electrically conductive materials such as metals including platinum, copper, silver, palladium, gold, tungsten, as well as combinations comprising at least one of the foregoing.

Disposed between the insulating layers 34 and 36 can be an electromagnetic shield 42. The electromagnetic shield 42 isolates electrical influences by dispersing electrical interferences and creating a barrier between a high power source (such as the heater) and a low power source (such as the temperature sensor and the gas sensing cell). The shield can comprise, for example, a line pattern (connected parallel lines, serpentine, cross hatch pattern, and/or the like). Some possible materials for the shield can include those materials discussed above in relation to the heater 44.

At the sensing end 21 of the sensor element 10, the electrical leads 58, are disposed in physical contact and in electrical communication with electrodes 12, 14, 18, 20, 80, 82. Further, electrical leads 58 are disposed in electrical communication with the heater 44 and the electromagnetic shield 42. Each electrical lead extends from a contact pad or via toward the sensing end 21. Electrical leads not disposed on a top surface or a bottom surface of the sensor element 10 are in electrical communication with the contact pads through vias formed in the layers. Two sets of three contact pads are disposed at the terminal end 81 of the sensor element 10: the first, second, and third contact pads 60, 70, 76 are disposed on the upper surface of the sensor element 10, and the fourth, fifth and sixth contact pads 62, 90, 92 are disposed on the lower surface of the sensor element 10. The first, second, third, and fourth contact pads 60, 62, 70, 76 are in electrical communication with a processor (not shown), and the fifth and sixth contact pads are in electrical communication with an external power source (not shown).

The insulating layers 22, 24, 28, 34, 36, 38 can comprise a dielectric material such as alumina, and the like. Each of the insulating layers can comprise a sufficient thickness to attain the desired insulating and/or structural properties. For example, each insulating layer can have a thickness of up to about 200 micrometers or so, depending upon the number of layers employed, or, more specifically, a thickness of about 50 micrometers to about 200 micrometers. Further, the sensor element 10 can comprise additional insulating layers to isolate electrical devices, segregate gases, and/or to provide additional structural support.

The active layers 26, 30, and 32 can comprise material that, while under the operating conditions of sensor element 10, is capable of permitting the electrochemical transfer of oxygen ions. These include the same or similar materials to those described as comprising electrolyte layer 16. Each of the active layers can comprise a thickness of up to about 200 micrometers or so, depending upon the number of layers employed, or, more specifically, a thickness of about 50 micrometers to about 200 micrometers.

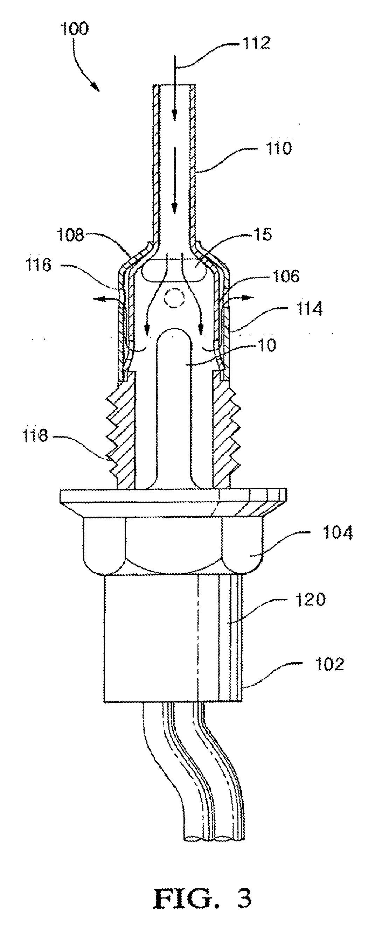

Referring to FIG. 3, a sensor 100 can be used for placement in a gas stream. Sensor element 10 can be disposed within a protective casing 120. The protective casing 120 can comprise an outer shield 108 having a plurality of outer shield holes 116. An inner shield 106 has a plurality of passages 114, which allows fluid to enter a space between the inner shield 106 and the outer shield 108. Outer shield holes 116 allow fluid in the space between inner shield 106 and outer shield 108 to exit the casing 120. An optional sampling tube having an inlet 112 extends from the outer shield 108. The sampling tube opens into a catalyst surrounding sensor element 10. Arrows are shown to illustrate the general fluid flow direction within the protective casing.

The plurality of exhaust passages 114 can be disposed through inner shield 106 to allow the exhaust fluid a sufficient time to contact the sensor element 10 prior to exiting the protective casing 120. The plurality of exhaust passages 114 can be any size or shape sufficient to allow the passage of exhaust fluid.

Suitable materials for the protective casing 120 can include material that is capable of resisting under-car salt, temperature, and corrosion. For example, ferrous materials are employed such as ferritic stainless steels. Ferritic stainless steels can include stainless steels such as SS-409, SS-316, and the like.

As illustrated in FIG. 3, a catalyst 15 can be disposed in the exhaust stream, upstream from the first sensing cell 12/16/14. The catalyst 15 can comprise a material capable of converting hydrocarbons, carbon monoxide, ammonia, and/or hydrogen into water, nitrogen, and/or carbon dioxide. In one embodiment, the catalyst 15 comprises a material that, under the operating conditions of sensor element 10, is capable of efficiently converting NO to NO₂. In another embodiment, the catalyst 15 can comprise a material that, under the operating conditions of the sensor element 10, is capable of converting NO₂ to NO. The catalyst 15 can comprise a material including platinum, platinum alloys, and the like, as well as combinations comprising at least one of the foregoing. The catalyst 15 can further comprise a zeolite (e.g., alumina-silica zeolite powder).

The catalyst 15 can be disposed proximate various locations in the casing 120. In general, the catalyst 15 can be disposed at a location in which the sample gas can sufficiently contact the catalyst 15 upstream from the sensor element 10. For example the catalyst 15 can be disposed proximate the sampling tube 110 or can be disposed proximate the inner surface of the inner shield 106. The catalyst 15 can also be disposed outside the casing 120 upstream from the sensor element 10. For example, the catalyst 15 can be part of a catalyst bed reactor, upstream from the inlet 112 of the casing 120. In an exemplary embodiment, the sensor element 10 is disposed in an exhaust stream in fluid communication with engine exhaust. In addition to NH₃, O₂, and NOx, the sensor's operating environment includes other combustion by-products, for example, hydrocarbons, hydrogen, carbon monoxide, water, sulfur, sulfur-containing compounds, and/or combustion radicals (such as hydrogen and hydroxyl ions), and the like.

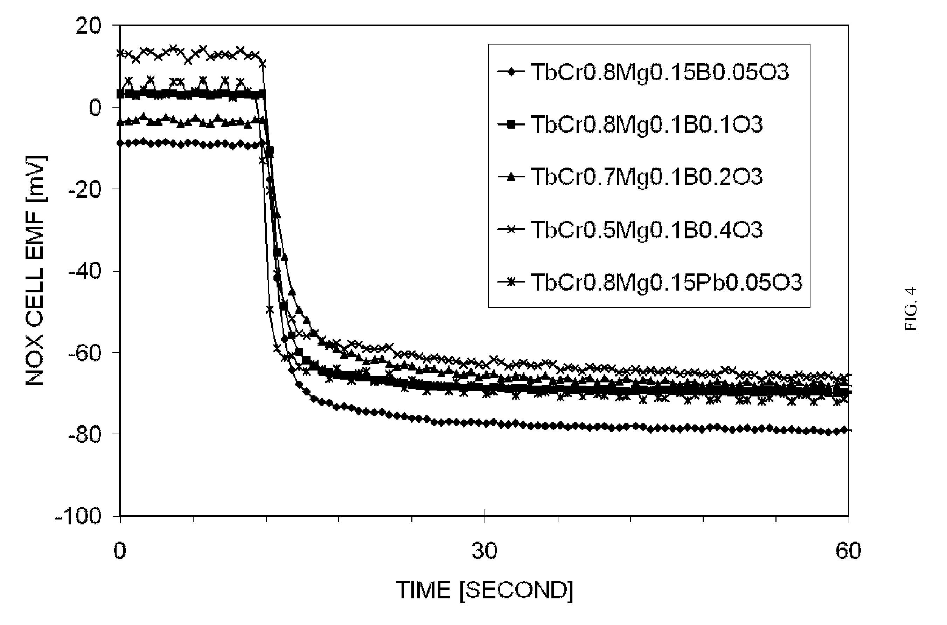

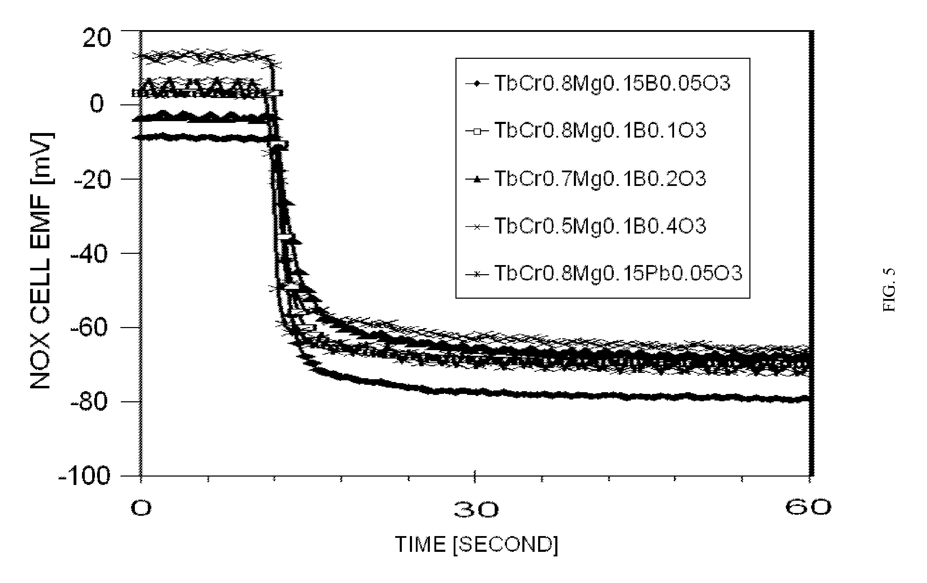

Referring now to FIG. 4 and FIG. 5, the response time of NOx sensors comprising five materials according to Formula (I) was measured. The materials were TbCr_{0.8}Mg_{0.15}B_{0.05}O₃, TbCr_{0.8}Mg_{0.1}B_{0.1}O₃, TbCr_{0.7}Mg_{0.1}B_{0.2}O₃, TbCr_{0.5}Mg_{0.1}B_{0.4}O₃, and TbCr_{0.8}Mg_{0.15}Pb_{0.05}O₃. They were prepared by comminuting a solid state mixture of the oxide of each elemental component, and heating the mixture for 6 hours at 1200° C. The amount of the oxide used to prepare the material can be determined according to the molar ratio of the non-oxide elements in the final composition. For example, TbCr_{0.8}Mg_{0.15}B_{0.05}O₃ can be prepared from Tb₃O₄, Cr₂O₃, MgO, and B₂O₃ in a molar ratio of 1:1.2:0.45:0.075 respectively, which is the equivalent of 1:0.8:0.15:0.025 molar ratio of the elements Tb, Cr, Mg, and B respectively.

The response time was measured between 20 parts per million (ppm) and 200 ppm of NO₂, with the background gas comprising 10 percent by volume O₂, 10 ppm NH₃, 1.5% H₂O, with the balance being N₂. In FIG. 4, the sensor was operated at a temperature of 570° C. In FIG. 5, the sensor was operated at a temperature of 531°C.

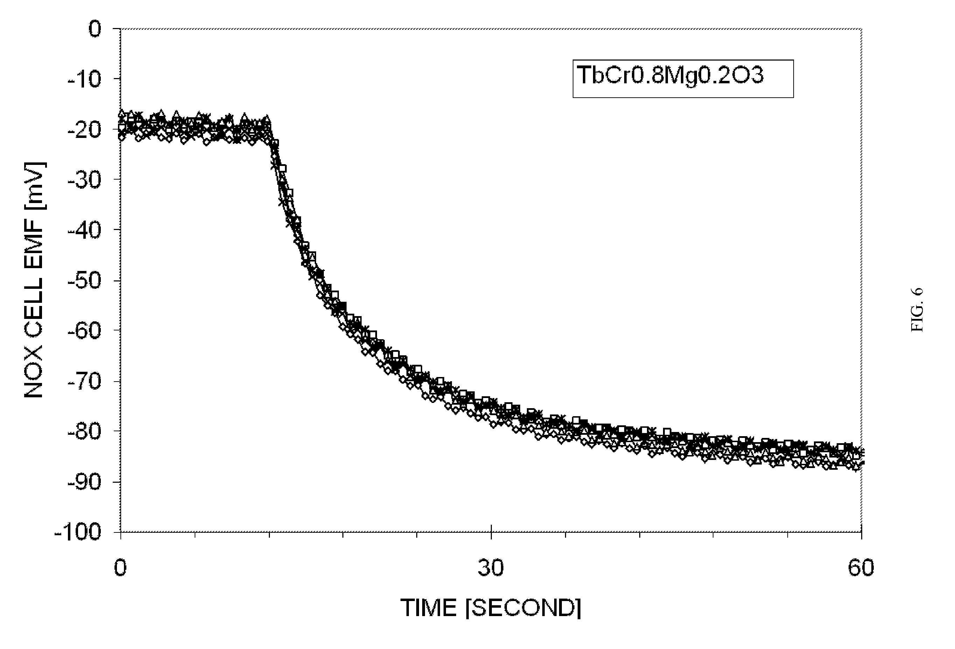

Referring to FIG. 6, the response time of NOx sensors comprising the material TbCr_{0.8}Mg_{0.2}O₃, which is not according to Formula (I), was measured. The sensor in FIG. 6 was operated at a temperature of 570° C, and the response time was measured between 20 ppm and 200 ppm of NO₂, with the background gas comprising 10 percent by volume O₂, 10 ppm NH₃, 1.5% H₂O, with the balance being N₂.

It can be seen that the response time exhibited by the sensors in FIG. 4 and FIG. 5 is substantially faster than that of the sensors in FIG. 6. For example, in FIG. 4, TbCr_{0.5}Mg_{0.1}B_{0.4}O₃ achieved an emf of -60 mV in about 12 seconds. On the other hand, TbCr_{0.8}Mg_{0.2}O₃ in FIG. 6 achieved an emf of -60 mV in about 22 seconds.

In addition, it can be seen that the time required for the sensor to reach the final emf measurement, that is, as graphically depicted in FIGS. 4-6, the time for the curve to reach a plateau, is much faster in FIG. 4 and FIG. 5 than it is in FIG. 6. For example, in FIG. 4, the time required for TbCr_{0.8}Mg_{0.15}B_{0.05}O₃ to exhibit an emf of about -75 mV going from about -9 mV, that is, the time between the two plateaus in the curve, is about 6 seconds. On the other hand, TbCr_{0.8}Mg_{0.2}O₃ in FIG. 6 achieves the same only in about 30 seconds.

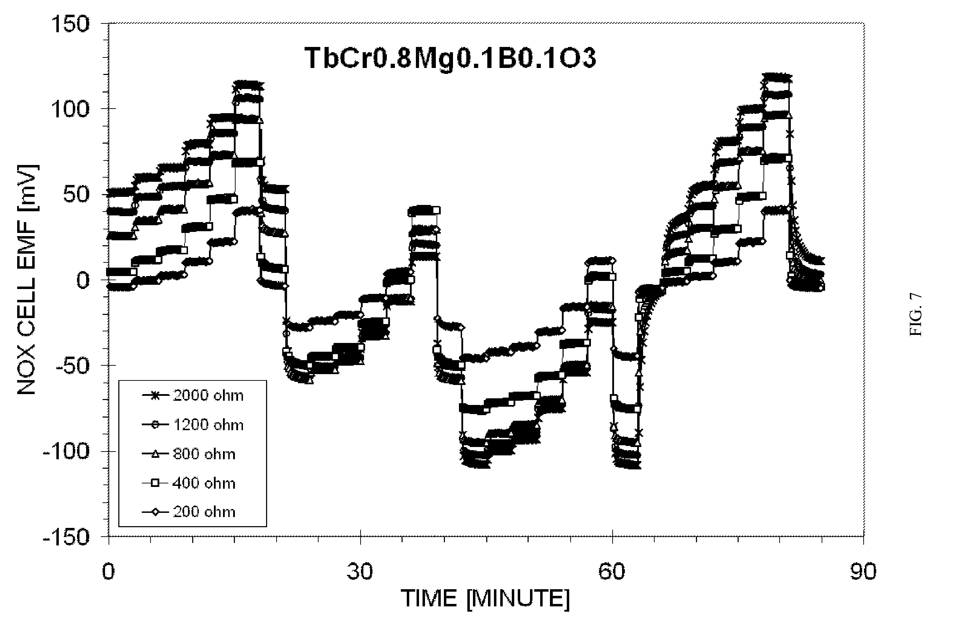

This sharp change in emf exhibited by the materials of Formula (I) is advantageous. This advantageous sensing behavior is achieved over a wide operational temperature range. Referring to FIG. 7, a graphical representation of the sensing behavior of a sensor comprising TbCr_{0.8}Mg_{0.1}B_{0.1}O₃, a material according to Formula (I), is illustrated. The sensor was controlled at 200, 400, 800, 1200, and 2000 ohms (Ω). A two-fold increase in the impedance value is indicative of an increase of about 30° C in temperature. It can be seen from the graphical representation illustrated in FIG. 7 that the time between two consecutive emf values at the resistance value is almost negligible, that is, the curve between two consecutive plateaus is essentially a vertical line.

### EXAMPLES 1-10

Examples 2-10 in Table 1 illustrate the average impedance and response time for several materials according to Formula (I), in addition to that of comparative example 1, wherein the material is TbCr_{0.8}Mg_{0.2}O₃.

**TABLE 1**

| Example | Formula | Impedance (Mega ohm) | Response time (sec) | emf at 200 ppm NO₂ (mV) |
|---|---|---|---|---|
| 1 | TbCr_{0.8}Mg_{0.2}O₃ | 1.4 | 19 | -87 |
| 2 | TbCr_{0.8}Mg_{0.15}B_{0.05}O₃ | 0.5 | 4 | -84 |
| 3 | TbCr_{0.8}Mg_{0.19}Pb_{0.01}O₃ | 0.8 | 3 | -81 |
| 4 | TbCr_{0.8}Mg_{0.15}P_{0.05}O₃ | 0.7 | 5 | -84 |
| 5 | Tb_{0.99}La_{0.01}Cr_{0.9}Mg_{0.175}B_{0.025}O₃ | 0.5 | 3 | -82 |
| 6 | TbCr_{0.8}Ba_{0.2}O₃ | 0.2 | 88 | -86 |
| 7 | TbCr_{0.8}Si_{0.2}O₃ | 0.2 | 45 | -59 |
| 8 | TbCr_{0.8}Mg_{0.1}B_{0.1}O₃ | 0.1 | not measured | not measured |
| 9 | TbCr_{0.7}Mg_{0.1}B_{0.2}O₃ | 0.2 | not measured | not measured |
| 10 | TbCr_{0.5}Mg_{0.1}B_{0.4}O₃ | 0.4 | not measured | not measured |

It can be seen from Table 1 that examples 2-10 have a substantially improved impedance value over comparative example 1. It can be further seen that examples 2-5 have a substantially improved response time over comparative example 1, in addition to the improved impedance. Examples 6 and 7 illustrate the pronounced advantageous effect caused by Ba and Si, which are represented by Z according to Formula (I), on the impedance of the material.

While the invention has been described with reference to an exemplary embodiment, it will be understood by those skilled in the art that various changes may be made and equivalents may be substituted for elements thereof without departing from the scope of the invention. In addition, many modifications may be made to adapt a particular situation or material to the teachings of the invention without departing from the essential scope thereof. Therefore, it is intended that the invention not be limited to the particular embodiment disclosed as the best mode contemplated for carrying out this invention, but that the invention will include all embodiments falling within the scope of the appended claims.

This written description uses examples and figures that are exemplary embodiments to disclose the invention, including the best mode, and also to enable any person skilled in the art to make and use the invention. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal language of the claims.

All ranges disclosed herein are inclusive of the endpoints, and the endpoints are independently combinable with each other. Further, it is understood that disclosing a range is specifically disclosing all ranges formed from any pair of any upper range limit and any lower range limit within this range, regardless of whether ranges are separately disclosed. It is not intended that the scope of the invention be limited to the specific values recited when defining a range.

The use of the terms "a" and "an" and "the" and similar referents in the context of describing the invention (especially in the context of the following claims) are to be construed to cover both the singular and the plural, unless otherwise indicated herein or clearly contradicted by context. Further, it should be noted that the terms "first," "second," and the like herein do not denote any order, quantity, or importance, but rather are used to distinguish one element from another. The modifier "about" used in connection with a quantity is inclusive of the stated value and has the meaning dictated by the context (e.g., it includes the degree of error associated with measurement of the particular quantity).

## Claims

1. A composition for use in a NOx electrode, comprising:
Tb₍₁₋ₓ₎Ln₍ₓ₎E_{(1-y)}Q_{(y1)}X_{(y2)}Z_{(y3)}O₃;
wherein:
Ln is a lanthanoid selected from the group consisting of lanthanum, cerium, praseodymium, neodymium, promethium, samarium, europium, gadolinium, dysprosium, holmium, erbium, thulium, ytterbium, and a combination thereof;
E is a metal selected from the group consisting of chromium, iron, and a combination thereof;
Q is an element selected from the group consisting of magnesium, calcium, strontium, and a combination thereof;
X is an element selected from the group consisting of boron, lead, phosphorus, germanium, and a combination thereof;
Z is an element selected from the group consisting of barium, silicon, aluminum, and a combination thereof;
x is from 0 to about 0.5;
y is from about 0.05 to about 0.8; and
y1, y2, y3 are independently from 0 to about 0.8;
with the proviso that:
y = y1 + y2 + y3; and
y2 + y3 is greater than 0.

2. The composition for use in a NOx electrode of claim 1, wherein:
E is chromium;
Q is an element selected from the group consisting of magnesium, strontium, and a combination thereof;
X is an element selected from the group consisting of boron, lead, phosphorus, and a combination thereof; and
Z is an element selected from the group consisting of barium, silicon, and a combination thereof.

3. The composition for use in a NOx electrode of claim 1, wherein x is greater than 0.

4. The composition for use in a NOx electrode of claim 1, wherein x is 0.

5. The composition for use in a NOx electrode of claim 1, wherein y1 is zero.

6. The composition for use in a NOx electrode of claim 1, wherein y1 is greater than 0.

7. The composition for use in a NOx electrode of claim 1, wherein y is from about 0.1 to about 0.3.

8. The composition for use in a NOx electrode of claim 1, wherein y is about 0.2.

9. The composition for use in a NOx electrode of claim 1, comprising TbCr0.8Mg0.15B0.05O3, TbCr0.8Mg0.1B0.1O3, TbCr0.7Mg0.1B0.2O3, TbCr0.5Mg0.1B0.4O3, TbCr0.8Mg0.19Pb0.01O3, TbCr0.8Mg0.15Pb0.05O3, TbCr0.8Mg0.15P0.05O3, Tb0.99La0.01Cr0.8Mg0.175B0.025O3, TbCr0.8Ba0.2O3, or TbCr0.8Si0.2O3.

10. A NOx electrode, comprising the composition for use in a NOx electrode of claim 1.

11. A NOx or NH3 sensor element comprising the NOx electrode of claim 10.

12. The NOx or NH3 sensor element of claim 11, further comprising:
a reference electrode (14); and
an electrolyte (16) disposed between the NOx electrode (12) and the reference electrode (14).

13. The NOx or NH3 sensor element of claim 12, further comprising:
a second NOx electrode (18);
a second reference electrode (20); and
an electrolyte (16) disposed between the second NOx electrode (18) and the second reference electrode (20).

14. The NOx or NH3 sensor element of claim 13, wherein the second NOx electrode (18) comprises the composition for use in a NOx electrode of claim 1.

15. A NOx or NH3 sensor comprising the NOx or NH3 sensor element of any one of claims 11 to 14.

16. A NOx or NH3 sensor of claim 15, further comprising:
a housing, wherein the NOx or NH3 sensor element (10) is disposed in the housing, and wherein the housing has an inlet for receiving a gas such that the gas can contact the sensor element (10); and
a catalyst upstream of the sensor element (10) such that:
the gas contacts the catalyst (15) prior to contacting the sensor element (10); and
hydrocarbons, carbon monoxide, ammonia, hydrogen, or a combination thereof, in the gas are converted to nitrogen, carbon dioxide, water, or a combination thereof.

17. A method of detecting NOx in a gas, comprising contacting the NOx electrode of claim 10 with the gas.

18. The method of detecting NOx in a gas of claim 17, further comprising:
determining a NOx emf between the NOx electrode (12) and a first reference electrode (14); and
determining a NOx concentration using the NOx emf.

19. The method of detecting NOx in a gas of claim 18, further comprising:
contacting a second NOx electrode (18) with the gas;
determining a NOx emf between the second NOx electrode (14) and a second reference electrode (20); and
determining a NOx concentration using the NOx emf determined between the first NOx electrode (12) and a first reference electrode (14) and/or between the second NOx electrode (14) and a second reference electrode (20).

20. The method of any one of claims 17-19, wherein the gas is an exhaust gas produced by an internal combustion engine.

21. The method of claim 20, wherein the engine is in an automobile.

22. A method of making the composition for use in a NOx electrode of claim 1, comprising heating a mixture of an oxide of:
Tb, E, and X;
Tb, E, and Z; or
Tb, E, X, and Z;
and, optionally, an oxide of:
Ln;
Q; or
Ln and Q;
at a temperature of about 500 °C to about 1600 °C.

23. The method of claim 22, wherein heating is for about 0.5 to about 48 hours.
